Europäisches Patentamt
European Patent Office
Office européen des brevets

(19)

(11) Publication number: **0 054 986 A1**

# EUROPEAN PATENT APPLICATION

(12)

(21) Application number: 81201265.6

(22) Date of filing: 12.11.81

(51) Int. Cl.³: C 07 C 45/50, C 07 C 47/02, C 07 C 47/225, C 07 C 47/11

(30) Priority: 22.12.80 GB 8041098

(43) Date of publication of application: 30.06.82 Bulletin 82/26

(84) Designated Contracting States: BE DE FR GB IT NL

(71) Applicant: SHELL INTERNATIONALE RESEARCH MAATSCHAPPIJ B.V., Carel van Bylandtlaan 30, NL-2596 HR Den Haag (NL)

(72) Inventor: Van Leeuwen, Petrus Wilhelmus N. M., Badhuisweg 3, NL-1031 CM Amsterdam (NL)
Inventor: Roobeek, Cornelis Franciscus, Badhuisweg 3, NL-1031 CM Amsterdam (NL)

(74) Representative: Pulster, Antonius Tonnis, Mr. et al, P.O. Box 302, NL-2501 CH The Hague (NL)

(54) **A process for the hydroformylation of olefins.**

(57) A process for the hydroformylation of less reactive olefins with hydrogen and carbon monoxide in the presence of a hydroformylation catalyst which has been modified by a ligand of formula $L(OR^1)(OR^2)(OR^3)$, wherein L represents P or As and $R^1$, $R^2$ and $R^3$ are (dis)similar aryl groups provided that at least one of $R^1$, $R^2$ and $R^3$ is a group

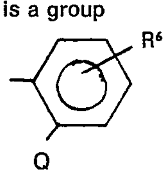

wherein Q represents a group 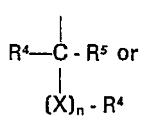 or

a group 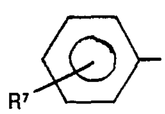,

wherein $H^4$ is an optionally fluorine-substituted hydrocarbyl group, $R^5$ is H or $R^4$, $R^6$ is H or an inert substituent on the meta and/or the para position of the ring, X is oxygen or sulphur, n is 0 or 1, and $R^7$ represents a hydrogen atom or an inert substituent. Less reactive olefins such as limonene can be hydroformylated with very good results.

EP 0 054 986 A1

# A PROCESS FOR THE HYDROFORMYLATION OF OLEFINS

The present invention relates to a process for the hydroformylation of olefins using a modified Group VIII-metal catalyst.

It is known that the hydroformylation reaction can be performed with good results using catalysts which comprise a Group VIII-metal compound which also contains a phosphorus or arsen moiety. In general, preference is given to the use of phosphorus compounds, especially of trialkyl or triarylphosphines or phosphites as ligands in the catalytic hydroformylation of olefins.

It should be noted, however, that there is a vast difference in reactivity between various olefins under similar reaction conditions using an identical catalytic system. It is also known that the organo-phosphorus ligands may exert a distinct influence on the selectivity of the hydroformylation reaction when the same olefin is applied as the substrate. For instance, it is known from the article by Pruett and Smith (J. Org. Chem., Vol. 34, (1969), No. 2, pp 307-330) that in the hydroformylation of 1-octene with trisubstituted phosphorus-containing ligands, an aliphatic substituent gives a lower percentage of straight-chain (or normal) aldehyde than an aromatic substituent. However, the selectivity between various aromatic ligands, phosphines as well as phosphites does not seem to differ markedly. From German Auslegeschrift 2039938 it appears that the hydroformylation of 1-hexane using a rhodium catalyst modified with various aromatic phosphites gives about the same yield and selectivity whereas the conversion using a catalyst comprising the unsubstituted aromatic phosphite, i.e. triphenyl phosphite, is at least 10% higher.

It has now been found that a certain class of olefins which can be regarded as markedly less reactive olefins compared with olefins such as 1-octene and 1-hexene can be hydroformylated at high rates and with good selectivity using Group VIII-metal catalysts modified with specific aromatic phosphites.

The present invention therefore relates to a process for the hydroformylation of olefins which comprises reacting an olefin containing the group 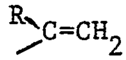 or the group 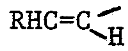 wherein R represents a hydrocarbyl group and the valence bond shown forms part of a hydrocarbyl group, or R together with the valence bond shown represents a ring structure having at least 5 carbon atoms in the ring, with carbon monoxide and hydrogen in the presence of a hydroformylation catalyst which has been modified by a ligand according to the general formula

$$L(OR^1)(OR^2)(OR^3)$$

wherein L represents a phosphorus or arsen moiety and $R^1$, $R^2$ and $R^3$ represent (dis)similar aryl groups provided that at least one of $R^1$, $R^2$ and $R^3$ represents a group

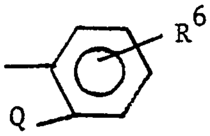 wherein Q represents a group 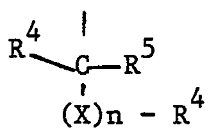

or a group 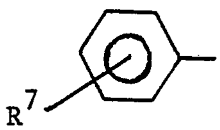

wherein each $R^4$ which may be the same or different represents an optionally fluorine containing hydrocarbyl group, $R^5$ represents a hydrogen atom or a group $R^4$, and $R^6$ represents a hydrogen atom or an inert substituent on the meta and/or para position of the ring, whilst X represents an oxygen or sulphur atom and n in 0 or 1, and $R^7$ represents a hydrogen atom or an inert substituent.

Examples of a class of olefins containing a group 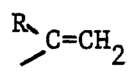 or 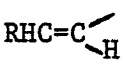 in the molecule which can be suitably used as starting materials in the process according to the present invention comprise β-alkyl-substituted acyclic α-olefins such as 2-methyl-1-butene, 2-methyl-1-pentene, 2-methyl-1-hexene, 2-ethyl-1-hexene, 2,3-dimethyl-1-butene, 2,3-dimethyl-1-pentene 2,3,3-trimethyl-1-butene, 2,3-dimethyl-1-hexene, 2,5-dimethyl-1-hexene, 2-ethyl-3-methyl-1-pentene, 2,6-dimethyl-1-heptene as well as β-alkyl-substituted α-olefins which contain a further β-substituted double bond such as 2,4-dimethyl-1,4-butadiene and isononadiene (2,6-dimethyl-1,5-heptadiene). The latter compound is

of interest as a starting material for citronellal. Particularly preferred as starting olefins are 2-methyl-1-hexene or 2-methyl-1-heptene.

Another class of olefins which can be used suitably comprises β-alkyl-substituted α-olefins which contain also a cyclic structure in the molecule which may or may not contain additional double bonds. Examples of such compounds comprise isopropenylcyclopentane, isopropenylcyclohexane, isobutenylcyclopentane, isobutenylcyclohexane, isopropenylbenzene (α-methylstyrene) and isobutenylbenzene as well as isoalkenyl-cycloalkenes provided the double bond in the ring structure comprises at least a lower alkyl group such as limonene (1-methyl-4-isopropenyl-1-cyclohexene). Also compounds comprising a further β-substituted double bond such as 1,4-diisopropenyl cyclohexane can be used. If desired the ring structures may be substituted by one or more inert substituents such as fluorine, chlorine or alkyl groups as well as hydroxy groups not connected to or neighbouring a carbon atom forming part of a double bond. Typical examples comprise 1-isopropenyl-4-chlorocyclohexane and plinol (1-isopropenyl-2,3-dimethyl-3-hydroxycyclopentane). Preferred as starting materials of this type are the isopropenylcycloalkanes and isopropenylcycloalkenes, especially limonene.

A further class of olefins which can be used suitably comprises cyclic olefins having either a methylene group in the molecule or an unsubstituted double bond in the molecule. Examples of such olefins comprise methylenecyclopentane, methylenecyclohexane, cyclopentene, cyclohexene, cyclooctene, 1,4-dihydronaphthalene, 1,2-dihydronaphthalene, 1,3-dihydroindane, 2,3-dihydroindane, 6,7-dihydro-5H-benzocycloheptene and 8,9-dehydro-5H-benzocycloheptene. Good results have been obtained using methylenecyclohexane and cyclohexene.

The process according to the present invention is carried out in the presence of a modified Group VIII-metal catalyst. Suitable Group VIII-metals comprise rhodium and iridium, preference being given to the use of rhodium as the Group VIII-metal in the

BAD ORIGINAL

hydroformylation catalyst. The amount of catalyst may vary within wide ranges, but generally the molar ratio of catalyst to olefin is in the range of from 1:10000 to 1:10, preferably between 1:5000 and 1:100.

The modified catalyst comprises a complex of a Group VIII-metal, carbon monoxide, an organic ligand, especially a phophite as defined hereinbefore and an anion to match the neutrality of the catalytic species. The catalyst can be prepared as such but it may also be formed in situ under the prevailing reaction conditions. Suitable starting materials comprise monovalent rhodium or iridium salts such as 1,5-cyclooctadienerhodium(I) acetate and 1,5-cyclooctadienerhodium(I) chloride. Chloride-containing starting materials are preferably first treated with a chloride acceptor.

As discussed hereinbefore, at least one of the groups $R^1$, $R^2$ and $R^3$ in the phosphite $L(OR^1)(OR^2)(OR^3)$ represents a specific ortho-substituted phenyl group. The nature of this group is rather critical with respect to the hydroformylation of less reactive olefins. It has been found that the use of tris(o-tolyl)phosphite gives only a very slow initial rate in the hydroformylation of 2-methyl-1-hexene comparable with the yield obtained by using the well-known ligand triphenylphosphine and that no product could be found at all when tris(2,6-dimethylphenyl)phosphite was used as the phosphorus ligand under the reaction conditions according to the present invention.

Preference is given to the use of phosphites according to the general formula $L(OR^1)(OR^2)(OR^3)$ wherein the groups $R^1$, $R^2$ and $R^3$ are identical, but dissimilar groups can also be used suitably. Examples of ortho-substituted phenyl groups which can be suitably used comprise those wherein $R^4$ represents a $C_{1-6}$ alkyl group, $R^5$ represents a hydrogen atom or a $C_{1-6}$ alkyl group, $R^6$ represents a hydrogen atom, a fluorine or chlorine atom, a $C_{1-20}$ alkyl or $C_{1-20}$ alkoxy group and n is 0 or 1 in which case X represents an oxygen or sulphur atom, and $R^7$ represents a hydrogen, fluorine or chlorine atom or a $C_{1-6}$ alkyl or $C_{1-6}$ alkoxy group. Preference is given to the presence of ortho-substituted phenyl groups wherein $R^4$

represents a methyl or ethyl group, $R^5$ represents a hydrogen atom or a methyl group, $R^6$ represents a hydrogen atom or a methyl, ethyl or octyl group, n is 0, and $R^7$ represents a hydrogen atom. Very good results have been obtained using tris(2-t.butylphenyl) phosphite or tris(2-t.butyl-4-methylphenyl) phosphite as the phosphorus ligand in the hydroformylation of various less reactive olefins, especially cyclohexene, limonene and methylenecyclohexane.

The process according to the present invention is preferably conducted in the presence of an excess of the phosphite having the general formula $L(OR^1)(OR^2)(OR^3)$ since such excess exerts a beneficial effect on the catalytic activity. Normally, metal-phosphorus ratios of from 1:2 to 1:100 can be used, preference being given to ratios in the range of from 1:4 to 1:20.

Generally the hydroformylation of the olefins as defined hereinbefore is carried out under rather mild conditions. Temperatures in the range of from 50°C to 200°C can be suitably applied, whilst temperatures in the range of from 80°C to 130°C are preferred.

The pressure, which is the sum of the hydrogen and carbon monoxide pressure, is suitably in the range of from 200 to 5000 kPa, preferably between 300 and 3500 kPa, although lower or higher pressures are not excluded. Generally the molar ratio of hydrogen to carbon monoxide is in the range of from 1:2 to 12:1. Good results have been obtained using hydrogen and carbon monoxide in equimolar as well as in 2:1 molar ratios.

The process according to the present invention may be conducted in the presence of an inert solvent. Examples of suitable solvents comprise saturated hydrocarbons such as pentane, hexane and higher alkanes, naphtha, kerosene, mineral oil, cyclopentane and cyclohexane, as well as the aromatic hydrocarbons such as benzene, toluene and the xylenes as well as ethers, ketones and nitriles. Also mixtures of solvents can be suitably applied. It is also possible to use an excess of the starting material as solvent.

The process according to the invention can be readily carried out using well-known chemical engineering practice which includes

continuous, semi-continuous and batch operation. The reaction time may vary between wide limits, from a couple of minutes to several hours depending on the specific olefin and phosphite applied. After the reaction the reaction mixture is worked up by techniques known in the art. The product aldehyde can be recovered by various means, e.g. by distillation. It is also possible to recycle part or all of the catalyst and the remaining reaction mixture.

The invention will now be illustrated by means of the following Examples.

EXAMPLE 1

A 100 ml stainless steel autoclave was charged with 20 ml benzene, in which were dissolved 0.02 mmol of 1,5-cyclooctadiene-rhodium(I) acetate and 0.2 mmol of tris(2-t.butylphenyl) phosphite, under an atmosphere of argon and the autoclave was also charged with 10 mmol of 2-methyl-1-hexene. The autoclave was then pressurized with a 1:1 molar mixture of hydrogen and carbon monoxide and heated to 80°C, the pressure in the autoclave now being 1400 kPa. The autoclave was kept 1 h at the reaction temperature of 80°C and then cooled to ambient temperature. After venting the gases, the reaction product was analysed using gaschromatography and NMR. The product obtained contained the desired linear aldehyde 3-methylheptanal for more than 95%. The rate of the hydroformylation of this less reactive 2-methyl-1-hexene, expressed (as hereinafter) in moles per mole rhodium per h amounted to 1000.

EXAMPLE 2

The experiment described in the previous Example was repeated using a hydrogen:carbon monoxide ratio of 2:1 at a reaction temperature of 70°C and a total pressure of 1800 kPa. The rate of the hydroformylation amounted to 1600. Again the desired aldehyde had been formed for more than 95%.

EXAMPLE 3

The experiment described in Example 1 was repeated 0.2 mmol of tris(o-isopropylphenyl) phosphite under otherwise similar conditions. The rate of hydroformylation amounted to 300.

COMPARATIVE EXAMPLE A

The experiment described in Example 1 was repeated but using 0.2 mmol of tris(2-tolyl) phosphite under otherwise similar conditions. The rate of hydroformylation amounted only to 30.

COMPARATIVE EXAMPLE B

The experiment described in Example 1 was repeated but using 0.2 mmol of tris(2,6-dimethylphenyl) phosphite under otherwise similar conditions. No hydroformylated product could be detected at all.

COMPARATIVE EXAMPLE C

The experiment described in Example 2 was repeated but using 0.2 mmol of the well-known ligand triphenylphosphine. The reaction temperature was 72°C. The rate of hydroformylation amounted only to 50.

EXAMPLE 4

The autoclave described in Example 1 was charged with 20 ml benzene in which were dissolved 0.02 mmol 1,5-cyclooctadienerhodium(I) acetate and 0.1 mmol of tris(2-t.butylphenyl) phosphite under an atmosphere of argon. Cyclohexene (15 mmol) was added. The autoclave was then pressurized with hydrogen and carbon monoxide in a 2:1 molar ratio and then heated to 70°C, the pressure in the autoclave now being 1800 kPa. After 1 h the reaction mixture was worked up in the manner described in Example 1. The rate of hydroformylation amounted to 660.

EXAMPLE 5

The experiment described in the previous Example was repeated using 0.2 mmol of tris(2-t.butylphenyl) phosphite. The reaction was carried out at a temperature of 90°C. The rate of hydroformylation amounted to 4000.

COMPARATIVE EXAMPLE D

The experiment described in Example 4 was repeated using varying amounts of triphenylphosphine (from 0.01 up to 0.08 mmol). The rate of hydroformylation was always less than 25.

EXAMPLE 6

The autoclave described in Example 1 was charged with 20 ml

benzene, in which was dissolved 0.02 mmol 1,5-cyclooctadienerhodium(I) acetate and 0.3 mmol of tris(2-t.butylphenyl) phosphite under an atmosphere of argon. Limonene (12 mmol of a 1/1 mixture of the 1-methyl and 2-methyl isomers as obtained from dimerizing isoprene) was added. The autoclave was then pressurized with hydrogen and carbon monoxide in a 2:1 molar ratio and then heated to 90°C, the pressure in the autoclave being 1400 kPa. After 1 h the reaction mixture was worked up as described in Example 1. The rate of hydroformylation amounted to 3500.

EXAMPLE 7

The experiment described in the previous Example was repeated using 10 ml benzene and 0.2 mmol of tris(2-biphenylyl) phosphite under otherwise similar conditions. Limonene (10 ml) was added and the reaction proceeded under a pressure of 1000 kPa; the hydrogen:-carbon monoxide ratio being 2:1. The rate of hydroformylation amounted to 1500.

COMPARATIVE EXAMPLE E

The experiment described in Example 6 was repeated but using triphenylphosphine in rather large excess (0.4 mmol). The rate of hydroformylation amounted to 100.

EXAMPLE 8

The experiment described in Example 6 was repeated using natural limonene as the starting material (60 mmol) in half the amount of benzene in which the rhodium compound (0.03 mmol) had been dissolved together with 0.21 mmol of tris(2-t.butylphenyl) phosphite. The reaction temperature was 80°C. The rate of hydroformylation amounted to 1700.

EXAMPLE 9

The experiment described in Example 1 was repeated using methylenecyclohexane as the starting material. The reaction was carried out at a temperature of 75°C and the total pressure amounted to 2000 kPa (hydrogen:carbon monoxide 1:1). The rate of hydroformylation amounted to 3000.

EXAMPLE 10

In a two litre reactor were placed limonene (900 g),

1,5-cyclooctadienerhodium(I) acetate (175 mg) and tris(2-t.butyl-4-methylphenyl) phosphite (17.0 g). A mixture of hydrogen and carbon monoxide (mol ratio 1:1) was introduced into the liquid whilst stirring, the pressure being kept at 1500 kPa. The reaction mixture was heated to 100°C. After 7 hours the reaction mixture was worked up. Both the limonene conversion and the selectivity to aldehyde were 90%. After removal of the product and the addition of fresh limonene to the reactor, the remaining catalytic solution could be used again.

## CLAIMS

1. A process for the hydroformylaton of olefins, which comprises reacting an olefin containing the group 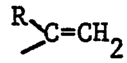 or the group 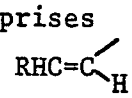 wherein R represents a hydrocarbyl group and the valence bond shown forms part of a hydrocarbyl group, or R together with the valence bond shown represents a ring structure having at least 5 carbon atoms in the ring, with carbon monoxide and hydrogen in the presence of a hydroformylation catalyst which has been modified by a ligand according to the general formula

$$L(OR^1)(OR^2)(OR^3)$$

wherein L represents a phosphorus or arsen moiety and $R^1$, $R^2$ and $R^3$ represent (dis)similar aryl groups provided that at least one of $R^1$, $R^2$ and $R^3$ represents a group

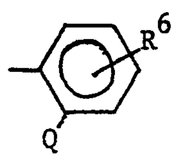 wherein Q represents a group 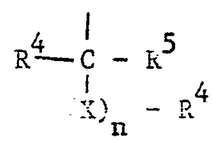

or a group 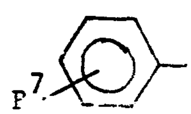

wherein each $R^4$ which may be the same or different represents an optionally fluorine-containing hydrocarbyl group, $R^5$ represents a hydrogen atom or a group $R^4$, and $R^6$ represents a hydrogen atom or an inert substituent on the meta and/or para position of the ring, whilst X represents as oxygen or sulphur atom and n is 0 or 1, and $R^7$ represents a hydrogen atom or an inert substituent.

2. A process according to claim 1, wherein a β-alkyl substituted acyclic α-olefin is used as the starting material.

3. A process according to claim 2, wherein 2 methyl-1-hexene is used as the starting material.

4. A process according to claim 1, wherein a β-alkyl substituted

BAD ORIGINAL

α-olefin containing also a cyclic substituent in the molecule is used as the starting material.

5. A process according to claim 4, wherein limonene is used as the starting material.

6. A process according to claim 1 wherein a cyclic olefin having either a methylene group or an unsubstituted double bond in the molecule is used as the starting material.

7. A process according to claim 6, wherein methylenecyclohexane is used.

8. A process according to claim 6, wherein cyclohexene is used.

9. A process according to any one of the preceding claims wherein a modified Group VIII-metal catalyst, preferably a rhodium catalyst is used.

10. A process according to claim 9, wherein a modified rhodium catalyst is used.

11. A process according to claim 9 or 10, wherein a catalyst is used which has been modified by a phosphite according to the general formula $L(OR^1)(OR^2)(OR^3)$ wherein at least one of the groups $R^1$, $R^2$ and $R^3$ represents an ortho-substituted phenyl group wherein $R^4$ represents a $C_{1-6}$ alkyl group, $R^5$ represents a hydrogen atom or a $C_{1-6}$ alkyl group, $R^6$ represents a hydrogen atom, a fluorine or chlorine atom, a $C_{1-20}$ alkyl or $C_{1-20}$ alkoxy group and n is 0 or 1 in which case X represents an oxygen or sulphur atom, and $R^7$ represents a hydrogen, fluorine or chlorine atom or a $C_{1-6}$ alkyl or $C_{1-6}$ alkoxy group.

12. A process according to claim 11, wherein a catalyst is used which has been modified by a phosphite containing an ortho-substituted phenyl group wherein $R^4$ represents a methyl or ethyl group, $R^5$ represents a hydrogen atom or a methyl group, $R^6$ represents a hydrogen atom or a methyl, ethyl or octyl group and n is 0, and $R^7$ represents a hydrogen atom.

13. A process according to claim 12, wherein a catalyst is used which has been modified by tris(2-t.butylphenyl) phosphite or tris(2-t.butyl-4-methylphenyl) phosphite.

14. A process according to any one of claims 11-13, wherein the phosphite is used in a metal:phosphorus ratio of from 1:2 to 1:100.

15. A process according to claim 15, wherein a metal: phosphorus ratio between 1:4 and 1:20 is used.

16. A process according to any one of the preceding claims, wherein the process is carried out at a temperature in the range of from $50^{o}C$ to $200^{o}C$.

17. A process according to claim 12, wherein a temperature between $80^{o}C$ and $130^{o}C$ is used.

18. A process according to any one of the preceding claims, wherein the process is carried out under a pressure between 300 and 3500 kPa.

19. A process as claimed in any one of the preceding claims, wherein a molar ratio of catalyst to olefin between 1:5000 and 1:100 is used.

20. A process as claimed in any one of the preceding claims, substantially as hereinbefore described with particular reference to the numbered Examples.

21. Products whenever prepared by a process as claimed in any one of claims 1 to 20.

0054986

European Patent Office

# EUROPEAN SEARCH REPORT

Application number

EP 81 20 1265

| DOCUMENTS CONSIDERED TO BE RELEVANT | | | CLASSIFICATION OF THE APPLICATION (Int. Cl. 3) |
|---|---|---|---|
| Category | Citation of document with indication, where appropriate, of relevant passages | Relevant to claim | |
| DX | THE JOURNAL OF ORGANIC CHEMISTRY vol. 34, no. 2, February 1969, pages 327-330 R.L. PRUETT et al.: "A Low-Pressure system for producing normal aldehydes by hydroformylation of α -olefins" * Page 328, table IV, example 7 * | 1,2,9-12,16-18 | C 07 C 45/50 47/02 47/225 47/11 |
| X | FR - A - 1 576 072 (UNION CARBIDE) * Claims, page 21, example 47 * | 1,2,9-12,16-18 | |
| A | US - A - 3 907 847 (KESTUTIS A. KEBLYS) * The whole document * | 1 | |

TECHNICAL FIELDS SEARCHED (Int.Cl. 3)

C 07 C 45/00
47/00

CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons
&: member of the same patent family, corresponding document

X The present search report has been drawn up for all claims

| Place of search | Date of completion of the search | Examiner |
|---|---|---|
| The Hague | 30-03-1982 | BONNEVALLE |

EPO Form 1503.1 06.78